# EUROPEAN PATENT SPECIFICATION

(11) **EP 1 795 208 B1**
(45) Date of publication and mention of the grant of the patent: **27.07.2011**
(21) Application number: 06025573.4
(22) Date of filing: 11.12.2006
(51) Int. Cl.: A61K 49/00, A61K 49/18, A61K 51/12, C07F 9/10, C07F 9/6524

(54) **Glycerophosphoric acid ester derivative having polyfunctional metal chelate structure**
Glycerophosphorsäureesterderivat mit polyfunktioneller Metallchelatstruktur
Dérivé d'ester d'acide glycérophosphorique ayant une structure chélatée métallique polyfonctionnelle

(30) Priority: 09.12.2005 JP 2005355493
(43) Date of publication of application: 13.06.2007
(73) Proprietor: Fujifilm Corporation, Minato-ku Tokyo 106-0031 (JP)
(72) Inventor: Takahashi, Kazunobu, Minami-ashigara-shi Kanagawa 250-0193 (JP); Aikawa, Kazuhiro, Minami-ashigara-shi Kanagawa 250-0193 (JP); Nishigaki, Junji, Minami-ashigara-shi Kanagawa 250-0193 (JP)
(74) Representative: HOFFMANN EITLE

(56) References cited:
- WO-A2-2004/067483
- ALHAIQUE, FRANCO ET AL: "Solvent 1H NMRD study of biotinylated paramagnetic liposomes containing Gd-bis-SDA-DTPA or Gd-DMPE-DTPA" INORGANICA CHIMICA ACTA , 331, 151-157 CODEN: ICHAA3; ISSN: 0020-1693, 2002, XP002428466
- DATABASE CA [Online] CHEMICAL ABSTRACTS SERVICE, COLUMBUS, OHIO, US; SEN GUPTA, ANIRBAN ET AL: "RGD-modified liposomes targeted to activated platelets as a potential vascular drug delivery system" XP002428471 retrieved from STN Database accession no. 2005:90852 -& SEN GUPTA, ANIRBAN ET AL: "RGD-modified liposomes targeted to activated platelets as a potential vascular drug delivery system" THROMBOSIS AND HAEMOSTASIS , 93(1), 106-114 CODEN: THHADQ; ISSN: 0340-6245, 2005, XP002428468

## Description

### Field of the Invention

The present invention relates to a glycerophosphoric acid ester derivative having a polyfuctional metal chelate structure. Said compound can be used as a membrane component of a liposome, and the resulting liposome can be used as a contrast agent.

### Background Art

A major example of non-invasive method for diagnosing arteriosclerosis includes X-ray angiography. This method contrasts vascular flows by using a watersoluble iodine-containing contrast medium, and therefore, the method has a problem of difficulty in distinguishing pathological lesions from normal tissues. By applying the above method, only a pathological lesion where constriction progresses 50% or more can be detected, and it is difficult to detect a lesion before onset of attack of an ischemic disease.

Methods of detecting a disease by nuclear magnetic resonance tomography (MRI) using a contrast medium, which is kinetically distributed preferentially in arteriosclerotic plaques, have been reported in recent years. However, hematoporphyrin derivatives (see, Patent document 1) are pointed out to have a defect of, for example, dermal deposition and coloring of skin. Further, gadolinium complexes having a perfluorinated side chain (see, Non-patent document 1) are reported to be accumulated in lipid-rich plaques, and thus they are concerned to be accumulated in lipid-rich tissues and organs of living bodies, such as fatty livers, renal epitheliums, and tendons of muscular tissues.

From a view point of chemical compounds, compounds are known in which phosphatidylethanolamine (PE) and diethylenetriaminepentaacetic acid (DTPA) are bound via an amide bond (for example, Non-patent document 2), and liposomes using gadolinium complexes of such compounds are also reported (Non-patent document 3). However, since these complexes are hardly soluble, they have poor property of handling in liposome formation, due to said property, they are concerned to possibly cause a problem of accumulation and toxicity in vivo.
[Patent document 1] U.S. Patent No. 4,577,636
[Non-patent document 1] Circulation, 109, 2890 (2004)
[Non-patent document 2] Polymeric Materials Science and Engineering, 89, 148 (2003)
[Non-patent document 3] Inorganica Chimica Acta, 331, 151 (2002)

### Disclosure of the Invention

An object of the present invention is to provide a compound suitable for a liposome contrast medium for performing lesion-selective imaging.

The inventors of the present invention conducted various researches to achieve the foregoing object. As a result, they found that a glycerin ester derivative having a polyfunctional metal chelate structure, which is represented by the following general formula (I), had superior properties as a component of liposomes as a contrast medium for MRI. The present invention was achieved on the basis of the above finding.

The present invention provides a compound as specified in appended Claim 1. The compounds of the present invention as well as related compounds belong to a family of compounds, which is represented by the following general formula (I), or a salt thereof: 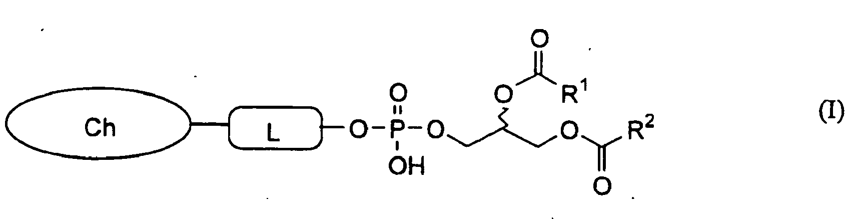 wherein R¹ and R² independently represent a substituted or unsubstituted alkyl or alkenyl group having 8 to 30 carbon atoms; L represents a divalent bridging group (L is constituted by atoms selected from the group consisting of carbon atom, oxygen atom, nitrogen atom and hydrogen atom, wherein the total number of atoms constituting L and selected from the group consisting of carbon atom, oxygen atom and nitrogen atom is 4 to 15); and Ch represents a chelate forming moiety containing 3 or more nitrogen atoms.

As a preferred embodiment of the aforementioned teaching, there is provided the aforementioned compound represented by the aforementioned general formula (I) or a salt thereof, which contains a structure represented by the following general formula (II) as a partial structure of Ch: 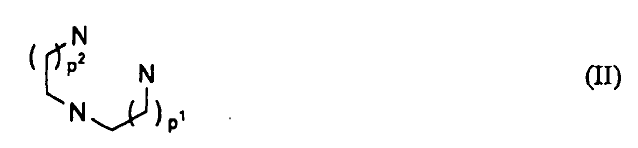 wherein p¹ and p² independently represent an integer of 1 or 2.

As more preferred embodiments of the aforementioned teaching, there are provided the aforementioned compound represented by the aforementioned general formula (I) or a salt thereof, which contains a structure represented by the following general formula (III) as a partial structure of Ch: 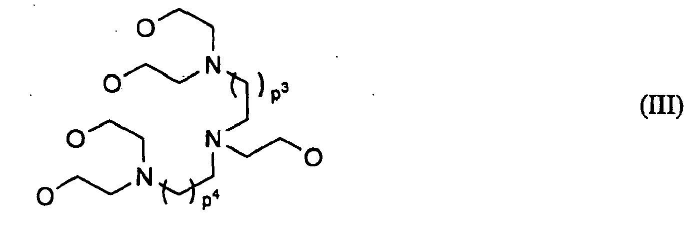 wherein p³ and p⁴ independently represent an integer of 1 or 2; and the aforementioned compound represented by the aforementioned general formula (I) or a salt thereof, which contains a structure represented by the following general formula (IV) as a partial structure of Ch: 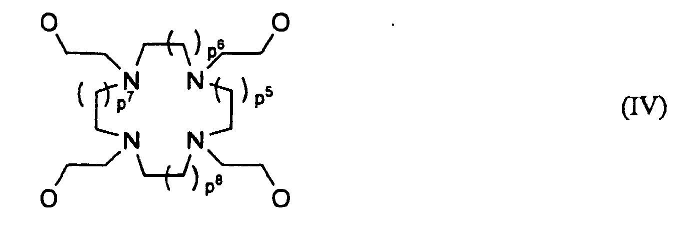 wherein p⁵, p⁶, p⁷, and p⁸ independently represent an integer of 1 or 2.

As a preferred embodiment of the aforementioned teaching, there is provided the compound represented by the aforementioned general formula (I) or a salt thereof, wherein the total number of atoms constituting L and selected from the group consisting of carbon atom, oxygen atom and nitrogen atom is 4 to 15, and the number of nitrogen atoms among them is 0 or 1. As a more preferred embodiment of the teaching, there is provided the compound represented by the aforementioned general formula (I) or a salt thereof, wherein L is a divalent bridging group represented as -X-CH₂CH₂(OCH₂CH₂)ₙ- wherein n represents an integer of 1 to 4, and -X- represents - O- or -NH-, or a divalent bridging group represented as -X-CH₂CH₂CH₂(OCH₂CH₂CH₂)ₘ- wherein m represents an integer of 0 to 2, and -X-represents -O- or -NH-.

As another preferred embodiment of the aforementioned teaching, there is provided the compound represented by the aforementioned general formula (I) or a salt thereof, wherein R¹ and R² independently represent an alkyl or alkenyl group having 10 to 20 carbon atoms.

The present invention also provides a chelate compound or a salt thereof, which consists of a compound as specified in appended claim 1 and a metal ion. As preferred embodiments of this invention, there are provided the aforementioned chelate compound as specified in appended claim 1 or a salt thereof, wherein the metal ion is a metal ion of an element selected from those of the atomic numbers 21 to 29, 31, 32, 37 to 39, 42 to 44, 49, and 57 to 83; and the aforementioned chelate compound as specified in appended claim 1 or a salt thereof, wherein the metal ion is a metal ion of a paramagnetic element selected from those of the atomic numbers 21 to 29, 42, 44, and 57 to 71.

From another aspect, the present invention provides a liposome containing the aforementioned compound as specified in appended claim 1 or a salt thereof as a membrane component, and according to a preferred embodiment thereof, there is provided the liposome containing a phosphatidylcholine and a phosphatidylserine as membrane components.

From a still further aspect of the present invention, there is provided a contrast medium for MRI comprising the aforementioned liposome. According to preferred embodiments of this invention, there are provided the aforementioned contrast medium for MRI, which is used for imaging of a vascular disease; the aforementioned contrast medium for MRI, which is used for imaging of vascular smooth muscle cells abnormally proliferating under influence of foam macrophages; the aforementioned contrast medium for MRI, which is used for imaging of a tissue or lesion in which macrophages localize; the aforementioned contrast medium for MRI, wherein the tissue in which macrophages localize is selected from the group consisting of tissues of liver, spleen, air vesicle, lymph node, lymph vessel, and renal epithelium; and the aforementioned contrast medium for MRI, wherein the lesion in which macrophages localize is selected from the group consisting of lesions of tumor, inflammation, and infection.

The present invention also provides a contrast medium for scintigraphy containing the aforementioned liposome. According to preferred embodiments of this invention, there are provided the aforementioned contrast medium for scintigraphy, which is used for imaging of a vascular disease; the aforementioned contrast medium for scintigraphy, which is used for imaging of vascular smooth muscle cells abnormally proliferating under influence of foam macrophages; the aforementioned contrast medium for scintigraphy, which is used for imaging of a tissue or lesion in which macrophages localize; the aforementioned contrast medium for scintigraphy, wherein the tissue in which macrophages localize is selected from the group consisting of tissues of liver, spleen, air vesicle, lymph node, lymph vessel, and renal epithelium; and the aforementioned contrast medium for scintigraphy, wherein the lesion in which macrophages localize is selected from the group consisting of lesions of tumor, inflammation, and infection.

Furthermore, the present teaching provides use of the aforementioned compound, chelate compound, or a salt of either of said compounds for the manufacture of the aforementioned contrast medium for MRI or contrast medium for scintigraphy; a method for MRI or scintigraphy imaging, which comprises the step of administering liposomes containing the aforementioned compound, chelate compound, or a salt of either of said compounds as a membrane component to a mammal including human; and a method for imaging a lesion of a vascular disease, which comprises the step of administering liposomes containing the aforementioned compound, chelate compound, or a salt of either of said compounds as a membrane component to a mammal including human, and then performing MRI or scintigraphy imaging.

The compound, chelate compound, and salt of either of said compounds according to the present invention can be used as a component lipid of liposomes for contrast medium for MRI or scintigraphy imaging, and have a property that they are efficiently incorporated in liposomes. A lesion of a vessel can be selectively contrasted by performing MRI or scintigraphy imaging using the liposomes containing the compound, chelate compound, or salt of either of said compounds according to the present invention.

### Brief Explanation of the Drawing

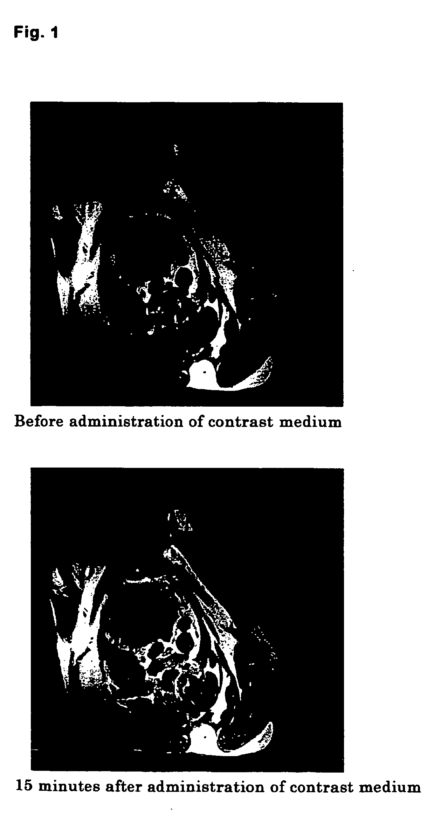

Fig. 1 depicts the result of MRI imaging of a rabbit with an already formed pathological legion in the aortic arch by using the contrast medium of the present invention.

### Best Mode for Carrying out the Invention

In the specification, when a functional group is referred to as "substituted or unsubstituted" or "may have a substituent", it is meant that the functional group may have one or more substituents. Unless otherwise specifically mentioned, the number, substituting position, and type of substituent to be bound are not particularly limited. When a certain functional group has two or more substituents, they may be the same or different. In the specification, when a certain functional group has a substituent, examples of the substituent include a halogen atom (in the specification, the "halogen atom" may be any of fluorine, chlorine, bromine, and iodine), an alkyl group (in the specification, the "alkyl group" include straight, branched, cyclic alkyl groups, and an alkyl group consisting of a combination thereof, and the cyclic alkyl group include a polycyclic alkyl group such as a bicycloalkyl group (the same shall apply to alkyl moieties of other substituents that contain the alkyl moieties)), an alkenyl group (including a cycloalkenyl group and a bicycloalkenyl group), an alkynyl group, an aryl group, a heterocyclic group, cyano group, hydroxyl group, nitro group, carboxyl group, an alkoxyl group, an aryloxy group, a silyloxy group, a heterocyclyloxy group, an acyloxy group, carbamoyloxy group, an alkoxycarbonyloxy group, an aryloxycarbonyloxy group, amino group (including anilino group), an acylamino group, aminocarbonylamino group, an alkoxycarbonylamino group, an aryloxycarbonylamino group, sulfamoylamino group, an alkyl- or arylsulfonylamino group, mercapto group, an alkylthio group, an arylthio group, a heterocyclylthio group, sulfamoyl group, sulfo group, an alkyl- or arylsulfinyl group, an alkyl- or arylsulfonyl group, an acyl group, an aryloxycarbonyl group, an alkoxycarbonyl group, carbamoyl group, an aryl- or heterocyclylazo group, imido group, phosphino group, phosphinyl group, phosphinyloxy group, phosphinylamino group, and silyl group.

Ch represents a chelate forming moiety containing 3 or more nitrogen atoms in the structure thereof. Ch may have a straight, branched, or cyclic structure, or a structure consisting of a combination thereof. Examples of the chelate forming moiety represented by Ch include but not limited to, for example, diethylenetriaminepentaacetic acid [DTPA] unit, 1,4,7,10-tetraazacyclododecane-1,4,7,10-tetraacetic acid [DOTA] unit, 1,4,8,11-tetraazacyclotetradecane-1,4,8,11-tetraacetic acid [TETA] unit, and the like.

Ch will be more specifically explained. It is more preferred that Ch contains a structure represented by the following general formula (II) as a partial structure of Ch: 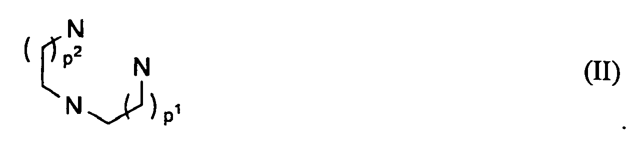 Ch is formed by adding arbitrary substituents to the partial structure represented by the aforementioned general formula (II), and the number, type and substitution position of the substituents are not particularly limited. L in the general formula (I) directly binds to any atom in the partial structure represented by the general formula (II). In the aforementioned general formula (II), p¹ and p² independently represent an integer of 1 or 2, and it is preferred that both represent 1.

Further, it is more preferred that Ch contains a structure represented by the following general formula (III): 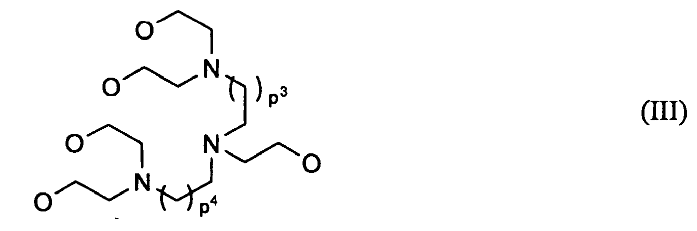 or the following general formula (IV): 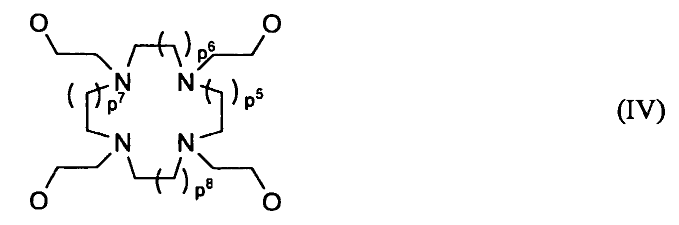 as a partial structure of Ch.

Ch is formed by adding arbitrary substituents to the partial structure represented by the aforementioned general formula (III) or (IV), and the number, type and substitution position of the substituents are not particularly limited. The number of the substituents is usually 20 or less, more preferably 15 or less, most preferably 10 or less. The partial structure represented by the general formula (III) or (IV) may contain a double bond. For example, the bond between carbon and carbon or carbon and oxygen may be a single bond or a double bond. When double bond is contained, although position and number of double bond are not particularly specified, the number is usually 10 or less, more preferably 5 or less. L in the general formula (I) directly binds to any atom in the partial structure represented by the aforementioned general formula (III) or (IV). In the aforementioned general formula (III), p³ and p⁴ independently represent an integer of 1 or 2, and it is preferred that both represent 1. In the aforementioned general formula (IV), p⁵, p⁶, p⁷, and p⁸ independently represent an integer of 1 or 2, and it is preferred that p⁵ to p⁸ all represent 1, or p⁵ and p⁷ represent 1, and p⁶ and p8⁴ represent 2.

L represents a divalent bridging group constituted by atoms selected from the group consisting of carbon atom, oxygen atom, nitrogen atom and hydrogen atom, wherein the total number of atoms constituting L and selected from the group consisting of carbon atom, oxygen atom and nitrogen atom is 4 to 15. L may optionally not contain any one or two kinds of atoms among carbon atom, oxygen atom, and nitrogen atom, and in such compounds, the total number of the atoms constituting L and selected from the group consisting of carbon atom, oxygen atom, and nitrogen atom means a total number of the remaining kinds of atoms constituting L (for example, when any oxygen atom is not contained in L, carbon atoms and nitrogen atoms). The total number of the atoms constituting L and selected from the group consisting of carbon atom, oxygen atom, and nitrogen atom is more preferably 4 to 12, most preferably 4 to 9. The number of nitrogen atoms constituting L is more preferably 0 or 1, most preferably 1. L may have a linear, branched or cyclic structure, or a structure consisting of a combination thereof. L may be saturated or may contain an unsaturated bond. When L contains an unsaturated bond, type, position, and number thereof are not particularly limited.

When L is a divalent bridging group represented as -X-CH₂CH₂(OCH₂CH₂)ₙ-wherein n represents an integer of 1 to 4, and -X- represents -O- or -NH-, n is more preferably 1 or 2, most preferably 1. Further, -X- is more preferably -NH-. When L is a divalent bridging group represented by -X-CH₂CH₂CH₂(OCH₂CH₂CH₂)ₘ- wherein m represents an integer of 0 to 2, and -X- represents -O- or -NH-, m is more preferably 0 or 1. Further, -X- is more preferably -NH-. When L is any of the aforementioned divalent bridging groups, L binds to the Ch group on the X side of L. Although L may have an arbitrary number of substituents, even in such compounds, L is constituted by atoms selected from the group consisting of carbon atom, oxygen atom, nitrogen atom, and hydrogen atom, and the total number of the atoms constituting L, selected from the group consisting of carbon atom, oxygen atom and nitrogen atom, is not out of the range of 1 to 15.

R¹ and R² independently represent an alkyl group or alkenyl group having 8 to 30 carbon atoms. The alkyl group or alkenyl group may be a straight, branched or cyclic alkyl group or alkenyl group, or an alkyl group or alkenyl group consisting of a combination thereof. However, the alkyl group or alkenyl group is preferably a straight or branched alkyl group or alkenyl group, most preferably a straight alkyl group or alkenyl group. Although the alkyl group or alkenyl group may have arbitrary type and number of substituents, an unsubstituted alkyl group or alkenyl group is preferred. The number of carbon atoms constituting R¹ or R² is more preferably 8 to 25, most preferably 10 to 20. When one or both of R¹ or R² represent an alkenyl group, double bond contained in the alkenyl group may be in E-configuration or Z-configuration, and the alkenyl group may be constituted by a mixture of groups in E-configuration and Z-configuration. When the alkenyl group is unsubstituted, a double bond in Z-configuration (cis-olefin) is more preferred. Although the number and position of double bond contained in the alkenyl group are not particularly limited, the number is preferably 5 or less, more preferably 3 or less.

The chelate compound of the present invention consists of the compound as specified in appended claim 1 and a metal ion. Although type of the metal ion is not particularly limited, metal ions of paramagnetic metals, heavy metals, and radioactive metals of radioactive metal isotopes are preferably used as metal ions suitable for the purpose of imaging by MRI, X-ray, ultrasonic contrast, scintigraphy and the like, or radiotherapy. More specifically, metal ions of elements selected from those of the atomic numbers 21 to 29, 31, 32, 37 to 39, 42 to 44, 49, and 57 to 83 are preferred. Examples of metal ions suitable for use of the chelate compound of the present invention as a contrast medium for MRI include metal ions of elements of the atomic numbers 21 to 29, 42, 44 and 57 to 71. For use in the preparation of positive MRI contrast medium, more preferred metals are those of the atomic numbers 24 (Cr), 25 (Mn), 26 (Fe), 63 (Eu), 64 (Gd), 66 (Dy), and 67 (Ho), those of the atomic numbers 25 (Mn), 26 (Fe), and 64 (Gd) are still more preferred, and Mn(II), Fe(III), and Gd(III) are especially preferred. For use in the preparation of negative MRI contrast medium, more preferred metals are those of the atomic numbers 62 (Sm), 65 (Tb), and 66 (Dy).

The compound and chelate compound of the present invention may have one or more asymmetric centers. In such compounds, stereoisomers such as optically active isomers and diastereomers based on the asymmetric centers may exist. Any of arbitrary stereoisomers in pure forms, arbitrary mixtures of stereoisomers, racemates and the like fall within the scope of the present invention. In the general formula (I), the bond represented by wavy line means that the carbon atom forming that bond is in S-configuration, R-configuration, or a mixture thereof, and the steric configuration of this carbon atom is not also particularly limited. Further, the compound described above may have one or more olefinic double bonds. The configuration thereof may be either E-configuration or Z-configuration, or the compound may be present as a mixture thereof. The compound of the present invention as specified in appended claim 1 may also exist as tautomers. Any tautomers or mixtures thereof fall within the scope of the present invention. Further, the compound as specified in appended claim 1 and chelate compound as specified in appended claims 2, 3 and 4 of the present invention may form a salt, and the compound in a free form and the compound in the form of a salt may form a hydrate or a solvate. All of these substances also fall within the scope of the present invention. Type of a salt is not particularly limited, and the salt may be an acid addition salt, or a base addition salt.

The compounds of the present invention as well as related compounds will be mentioned below.

| | | | |
|---|---|---|---|
| (1) | 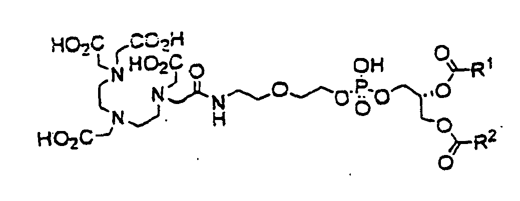 | 1) R¹=R²=n-C₈H₁₇ | |
| | | 2) R¹=R²=n-C₁₀H₂₁ | |
| | | 4) R¹=R²=n-C₁₃H₂₇ | |
| | | 5) R¹=R²=n-C₁₅H₃₁ | |
| | | 6) R¹=R²=n-C₁₇H₃₅ | |
| | | 7) R¹=R²=n-C₂₀H₄₁ | |
| | | 8) R¹=R²=n-C₂₅H₅₁ | |
| | | 9) R¹=R²=n-C₃₀H₆₁ | |
| | | 10) R¹=n-C₁₃H₂₇, R²=n-C₁₅H₃₁ | |
| (2) | 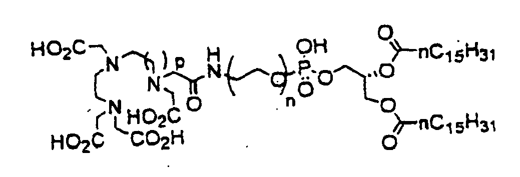 | 1) p=1, n=3 | 4) p=2, n=2 |
| | | 2) p=1, n=4 | 5) p=2, n=3 |
| | | 3) p=1, n=5 | 6) p=2, n=4 |
| | | | 7) p=2, n=5 |
| (3)* | 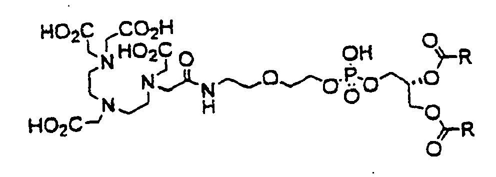 | 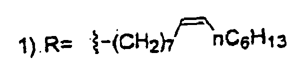 | |
| | | 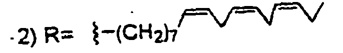 | |
| | | 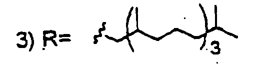 | |
| (4)* | 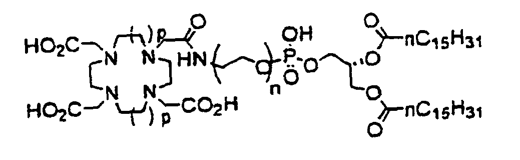 | 1) p=1, n=2 | 5) p=2, n=2 |
| | | 2) p=1, n=3 | 6) p=2, n=3 |
| | | 3) p=1, n=4 | 7) p=2, n=4 |
| | | 4) p=1, n=5 | 8) p=2, n=5 |
| (5)* | 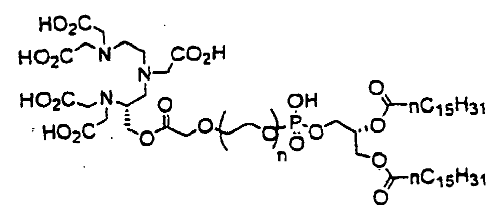 | 1) n=0 | |
| | | 2) n=1 | |
| | | 3) n=2 | |
| | | 4) n=3 | |
| (6)* | 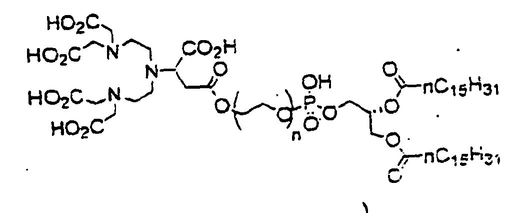 | 1) n=1 | |
| | | 2) n=2 | |
| | | 3) n=3 | |
| | | 4) n=4 | |
| (7)* | 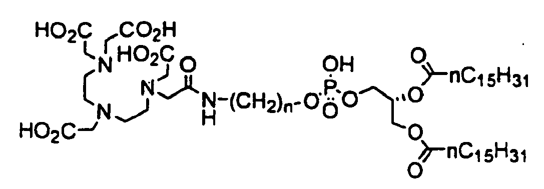 | 1) n=4 | |
| | | 2) n=6 | |
| | | 3) n=8 | |
| | | 4) n=10 | |
| (8)* | 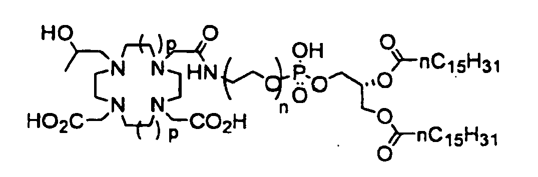 | 1) p=1, n=2 | 5) p=2, n=2 |
| | | 2) p=1, n=3 | 6) p=2, n=3 |
| | | 3) p=1, n=4 | 7) p=2, n=4 |
| | | 4) p=1, n=5 | 8) p=2, n=5 |
| (9)* | 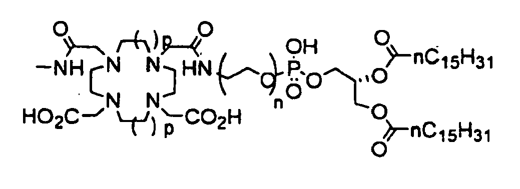 | 1) p=1, n=2 | 5) p=2, n=2 |
| | | 2) p=1, n=3 | 6) p=2, n=3 |
| | | 3) p=1, n=4 | 7) p=2, n=4 |
| | | 4) p=1, n=5 | 8) p=2, n=5 |
| (10)* | 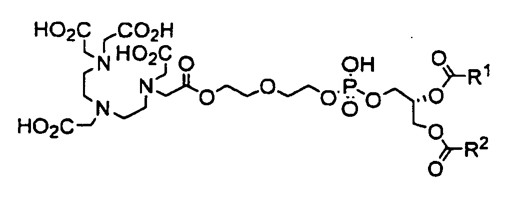 | 1) R¹=R²=n-C₈H₁₇ | |
| | | 2) R¹=R²=n-C₁₀H₂₁ | |
| | | 4) R¹=R²=n-C₁₃H₂₇ | |
| | | 5) R¹=R²=n-C₁₅H₃₁ | |
| | | 6) R¹=R²=n-C₁₇H₃₅ | |
| | | 7) R¹=R²=n-C₂₀H₄₁ | |
| | | 8) R¹=R²=n-C₂₅H₅₁ | |
| | | 9) R¹=R²=n-C₃₀H₆₁ | |
| | | 10) R¹=n-C₁₃H₂₇, R²=n-C₁₅H₃₁ | |
| (11)* | 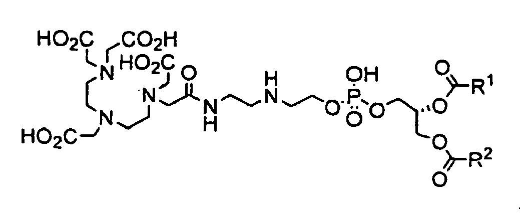 | 1) R¹=R²=n-C₈H₁₇ | |
| | | 2) R¹=R²=n-C₁₀H₂₁ | |
| | | 4) R¹=R²=n-C₁₃H₂₇ | |
| | | 5) R¹=R²=n-C₁₅H₃₁ | |
| | | 6) R¹=R²=n-C₁₇H₃₅ | |
| | | 7) R¹=R²=n-C₂₀H₄₁ | |
| | | 8) R¹=R²=n-C₂₅H₅₁ | |
| | | 9) R¹=R²=n-C₃₀H₆₁ | |
| | | 10) R¹=n-C₁₃H₂₇, R²=n-C₁₅H₃₁ | |
| (12)* | 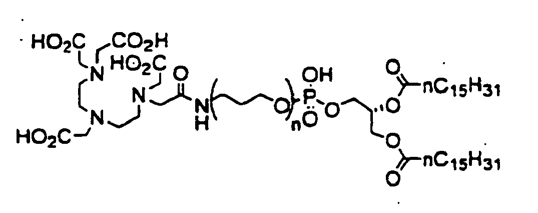 | 1) n=1 | |
| | | 2) n=2 | |
| | | 3) n=3 | |
| (13)* | 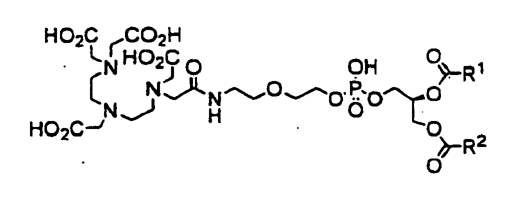 | 1) R¹=R²=n-C₈H₁₇ | |
| | | 2) R¹=R²=n-C₁₀H₂₁ | |
| | | 4) R¹=R²=n-C₁₃H₂₇ | |
| | | 5) R¹=R²=n-C₁₅H₃₁ | |
| | | 6) R¹=R²=n-C₁₇H₃₅ | |
| | | 7) R¹=R²=n-C₂₀H₄₁ | |
| | | 8) R¹=R²=n-C₂₅H₅₁ | |
| | | 9) R¹=R²=n-C₃₀H₆₁ | |
| | | 10) R¹=n-C₁₃H₂₇, R²=n-C₁₅H₃₁ | |
| (14)* | 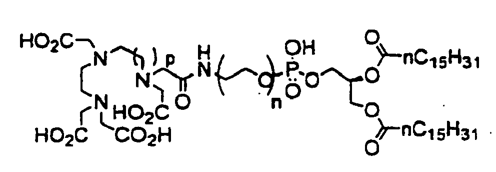 | 1) p=1, n=2 | 5) I=2, n=2 |
| | | 2) p=1, n=3 | 6) I=2, n=3 |
| | | 3) p=1, n=4 | 7) I=2, n=4 |
| | | 4) p=1, n=5 | 8) I=2, n=5 |
| (15)* | 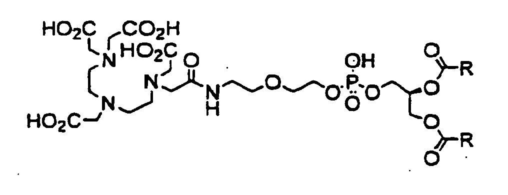 | 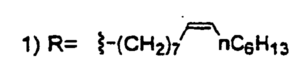 | |
| | | 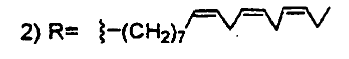 | |
| | | 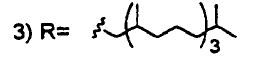 | |

| | | | |
|---|---|---|---|
| *(Comparative Compound) | | | |

Synthetic methods for the compound of the present invention, in general, will be explained. However, synthetic methods of the compound of the present invention are not limited to these methods. As the long chain fatty acids as a partial structure of the compound of the present invention, those ordinarily commercially available may be used, or they may be suitably synthesized depending on purposes. When they are obtained by syntheses, corresponding alcohols, alkyl halides and the like can be used as raw materials according to, for example, the method described by Richard C. Larock in Comprehensive Organic Transformations (VCH).

The aforementioned long chain fatty acids can be condensed with a derivative of glycerol, 1,3-dihydroxyacetone, derivatives thereof, or the like and thereby derived into a diacyl glyceride derivative. In this process, a protective group can also be used, if necessary. As a protective group used in such process, for example, any of the protective groups described by T.W. Green & P.G.M. Wuts in Protecting Groups in Organic Synthesis (John Wiley & Sonc, Inc.) can be suitably selected and used.

The aforementioned diacyl glyceride derivative can be suitably bound with a required bridging group and then bound with a polyamine derivative having a metal coordinating ability to synthesis the compound of the present invention. This process can be carried out according to the methods described in, for example, Bioconjugate Chem., 10, 137 (1999); Tetrahedron Lett., 37, 4685 (1996); J. Chem. Soc., Perkin Trans 2, 348 (2002); J. Heterocycl. Chem., 37, 387 (2000); Synthetic Commun., 26, 2511 (1996); J. Med. Chem., 47, 3629 (2004).

The compound of the present invention and a salt thereof can be used as a membrane component of a liposome. When a liposome is prepared by using the compound of the present invention or a salt thereof, amount of the compound of the present invention or a salt thereof is about from 5 to 90 mass %, preferably from 5 to 80 mass %, based on the total mass of membrane components. A single kind of the compound of the present invention, the chelate compound of the present invention, or a salt of one of these compounds may be used as the membrane component, or two or more kinds of said substances may be used in combination.

As other membrane components of liposome, any of lipid compounds ordinarily used for the preparation of liposomes can be used. Such compounds are described in, for example, Biochim. Biophys. Acta, 150 (4), 44 (1982); Adv. in Lipid. Res., 16 (1) 1 (1978); RESEARCH IN LIPOSOMES, P. Machy, L. Leserman, John Libbey EUROTEXT Co.; "Liposome", Ed., Nojima, Sunamoto and Inoue, Nankodo, and the like. As the lipid compounds, phospholipids are preferred, and phosphatidylcholines (PC) are particularly preferred. Preferred examples of phosphatidylcholines include egg PC, dimyristoyl-PC (DMPC), dipalmitoyl-PC (DPPC), distearoyl-PC (DSPC), dioleyl-PC (DOPC).

In a preferred embodiment of the present invention, a phosphatidylcholine and a phosphatidylserine (PS) are used in combination as membrane components. Examples of the phosphatidylserine include those having lipid moieties similar to those of the phospholipids mentioned as preferred examples of the phosphatidylcholines. When a phosphatidylcholine and a phosphatidylserine are used in combination, molar ratio of PC and PS (PC:PS) used is preferably in the range of 90:10 to 10:90, more preferably 30:70 to 70:30.

Another preferred embodiment of the liposome of the present invention includes a liposome containing a phosphatidylcholine and a phosphatidylserine and further containing a phosphoric acid dialkyl ester as membrane components. The two alkyl groups constituting the dialkyl ester of phosphoric acid dialkyl ester are preferably the same, and each alkyl group preferably contains 6 or more carbon atoms, more preferably 10 or more carbon atoms, still more preferably 12 or more carbon atoms. Preferred examples of the phosphoric acid dialkyl ester include, but not limited to, dilauryl phosphate, dimyristyl phosphate, dicetyl phosphate and the like. In this embodiment, preferred amount of the phosphoric acid dialkyl ester is from 1 to 50 mass %, more preferably from 1 to 30 mass %, still more preferably from 1 to 20 mass %, based on the total mass of phosphatidylcholine and phosphatidylserine.

In the liposome containing a phosphatidylcholine, a phosphatidylserine, a phosphoric acid dialkyl ester and the compound of the present invention as membrane components, preferred weight ratios of PC, PS, phosphoric acid dialkyl ester and the compound of the present invention is from 5 to 50 mass %: from 5 to 50 mass %: from 1 to 10 mass %: from 1 to 80 mass %.

The components of the liposome of the present invention are not limited to the aforementioned four kinds of compounds, and other components may be admixed. Examples of such components include cholesterol, cholesterol esters, sphingomyelin, monosial ganglioside GM1 derivatives described in FEBS Lett., 223, 42 (1987); Proc. Natl. Acad. Sci., USA, 85, 6949 (1988) etc., glucuronic acid derivatives described in Chem. Lett., 2145 (1989); Biochim. Biophys. Acta, 1148, 77 (1992) etc., and polyethylene glycol derivatives described in Biochim. Biophys. Acta, 1029, 91 (1990); FEBS Lett., 268, 235 (1990) etc.

The liposome of the present invention may be prepared by any methods available for those skilled in the art. Examples of the preparation methods are described in Ann. Rev. Biophys. Bioeng., 9, 467 (1980), "Liopsomes" (Ed. by M.J. Ostro, MARCELL DEKKER, INC.) and the like, as well as the published reviews of liposomes mentioned above. More specifically, examples include the ultrasonication method, ethanol injection method, French press method, ether injection method, cholic acid method, calcium fusion method, freeze and thawing method, reverse phase evaporation method and the like. Size of the liposome of the present invention may be any of those obtainable by the aforementioned methods. Generally, the size in average may be 400 nm or less, preferably 200 nm or less. Structure of the liposome is not also particularly limited, and may be any structure such as unilamellar or multilamellar structure. It is also possible to formulate one or more kinds of appropriate medicaments or other contrast media inside the liposome.

When the liposomes of the present invention are used as a contrast medium, the medium can be preferably administered parenterally, more preferably intravenously administered. For example, preparations in the form of an injection or a drip infusion can be provided as powdery compositions in a lyophilized form, and they can be used by being dissolved or resuspended just before use in water or an appropriate solvent (e.g., physiological saline, glucose infusion, buffering solution and the like). When the liposomes of the present invention are used as a contrast medium, the dose can be suitably determined so that the content of compounds in the liposomes becomes similar to that of a conventional contrast medium.

Although it is not intended to be bound by any specific theory, it is known that, in vascular diseases such as arteriosclerosis or restenosis after PTCA, vascular smooth muscle cells constituting tunica media of blood vessel abnormally proliferate and migrate into endosporium at the same time to narrow blood flow passages. Although triggers that initiate the abnormal proliferation of normal vascular smooth muscle cells have not yet been clearly elucidated, it is known that migration into endosporium and foaming of macrophages are important factors. It is reported that vascular smooth muscle cells then cause phenotype conversion (from constricted to composite type).

If the liposomes of the present invention are used, the compound serving as a defined contrast medium can be selectively taken up into the vascular smooth muscle cells abnormally proliferating under influences of foam macrophages. As a result, imaging becomes possible with high contrast between vascular smooth muscle cells of a lesion and a non-pathological site. Therefore, the contrast medium of the present invention can be suitably used particularly for MRI of vascular diseases. For example, imaging of arteriosclerotic lesion or restenosis after PTCA can be performed.

Further, as described in J. Biol. Chem., 265, 5226 (1990), for example, it is known that liposomes containing phospholipids, in particular, liposomes formed from PC and PS, are likely to accumulate on macrophages with the aid of scavenger receptors. Therefore, by using the liposomes of the present invention, the compound of the present invention can be accumulated in a tissue or a lesion in which macrophages localize. If the liposomes of the present invention are used, a predetermined compound can be accumulated in macrophages in a larger amount compared with the case of using suspension or oil emulsion belonging to known techniques.

Examples of tissues in which localization of macrophages is observed, which can be suitably imaged using the liposomes of the present invention, include blood vessel, liver, spleen, air vesicle, lymph node, lymph vessel, and renal epithelium. Further, it is known that macrophages accumulate in lesions in certain classes of diseases. Examples of such diseases include tumor, arteriosclerosis, inflammation, infection and the like. Therefore, lesions of such diseases can be identified by using the liposomes of the present invention. In particular, it is known that foam macrophages, which take up a large amount of denatured LDL with the aid of scavenger receptors, accumulate in atherosclerosis lesions at an early stage (Am. J. Pathol., 103, 181 (1981); Annu. Rev. Biochem., 52, 223 (1983)). Therefore, by performing imaging after accumulation of the liposomes of the present invention in the macrophages, it is possible to identify locations of atherosclerosis lesions at an early stage, which is hardly achievable by other means.

The imaging method using the liposomes of the present invention is not particularly limited. For example, imaging can be attained by measuring change in the T1/T2 relaxation time of water in the same manner as that in imaging methods using a usual contrast medium for MRI. Moreover, it is also possible to use them as a contrast medium for scintigraphy, X-ray contrast medium, optical image formation agent, and ultrasonic contrast agent by suitably using an appropriate metal ion.

### Examples

The present invention will be explained more specifically with reference to the following examples. The compound numbers used in the following examples correspond to the numbers of the examples of compounds mentioned above. The structures of the compounds mentioned in the examples were confirmed on the basis of NMR spectra and mass spectra.

### Synthesis of Compound 1-5

DMF (20 mL) and tetrahydrofuran (THF, 100 mL) were added to sodium hydride (3.44 g), and the mixture was stirred at 0°C. The mixture was added dropwise with a solution of (S)-(+)-2,2-dimethyl-1,3-dioxolane-4-methanol (10.4 g) in a mixture of DMF (10 mL) and THF (10 mL), and stirred at 0°C for 1 hour. The mixture was added with benzyl chloride (9.95 mL), and stirred at room temperature for 3 hours. The mixture was added with a saturated ammonium chloride solution, and extracted twice with ethyl acetate. The resulting organic layer was washed 4 times with water and once with saturated brine. The organic layer was dried over anhydrous magnesium sulfate, and then the solvent was removed to obtain a crude product of (S)-(+)-4-benzyloxymethyl-2,2-dimethyl-1,3-dioxolane (17.8 g, quantitative).

The crude product of (S)-(+)-4-benzyloxymethyl-2,2-dimethyl-1,3-dioxolane (17.0 g) was dissolved in methanol (60 mL), and the solution was added with 1 N hydrochloric acid (30 mL), and stirred at room temperature for 1 day. The reaction mixture was adjusted to pH 7 by adding saturated aqueous sodium hydrogencarbonate, and extracted 4 times with dichloromethane. The organic layer was dried over anhydrous sodium sulfate, and then the solvent was removed. The residue was purified by silica gel column chromatography to obtain (R)-(+)-3-benzyloxy-1,2-propanediol (11.8 g, yield: 87%).

(R)-(+)-3-Benzyloxy-1,2-propanediol (1.85 g) was dissolved in dichloromethane (10 mL), added with palmitic acid (5.64 g), dimethylaminopyridine (56 mg) and ethyldimethylaminopropylcarbodiimide hydrochloride (4.85 g), and the mixture was stirred at room temperature for 1 day. After the solvent was evaporated, the residue was purified by silica gel column chromatography to obtain benzyl-protected 1,2-diglyceride derivative (5.31 g, yield: 79%).

The aforementioned benzyl-protected compound (5.00 g) was dissolved in ethyl acetate (50 mL). The solution was added with 10% palladium/carbon (0.2 g), and the mixture was stirred for 1 day under a hydrogen atmosphere (5 MPa). After the catalyst was separated by filtration, the solvent was removed to obtain 1,2-dipalmitoyl glyceride (4.19 g, yield: 97%).
¹H-NMR (300MHz, CDCl₃) δ : 5.08 (1H, quin) 4.32 (1H, dd) 4.24 (1H, dd) 3.73 (2H, t) 2.33 (4H, q) 1.67-1.57 (4H, m) 1.33-1.22 (48H, m) 0.88(6H, t)

Ethylene glycol mono-2-chloroethyl ether (11.2 g) and phthalimide potassium salt (11.1 g) were dissolved in N,N-dimethylformamide (DMF, 10 mL), and the solution was stirred at 120°C for 2 hours. The reaction mixture was left to cool, then added with dichloromethane, and washed with 4 times with water and once with saturated brine. After the solvent was removed, the resulting residue was purified by silica gel column chromatography to obtain a phthalimidated alcohol (8.80 g, 62%). ¹H-NMR (300MHz, CDCl₃) δ : 7.90-7.82 (2H, m) 7.76-7.69 (2H, m) 3.92 (2H, t) 3.76 (2H, t) 3.72-3.65 (2H, m) 3.65-3.58 (2H, m) 2.30 (1H, t)

The above alcohol (2.11 g) was dissolved in dichloromethane (20 mL), and the solution was added with triethylamine (TEA, 2.76 g), and stirred at 0°C. The mixture was added with N,N-diisopropylmethylphosphoamidic chloride (2.30 mL) and stirred at room temperature for 10 minutes. Then, the mixture was added with ice-cooled saturated aqueous sodium hydrogencarbonate, and extracted twice with dichloromethane. After the organic layer was dried over sodium sulfate, the solvent was removed. The residue was added with dichloromethane (60 mL) and dissolved therein, and the solution was added with the aforementioned 1,2-dipalmitoyl glyceride (4.65 g) and tetrazole (0.91 g). The solution was stirred for 15 minutes, and then added with 31% aqueous hydrogen peroxide (4 mL), and stirring was continued for 20 minutes. The reaction mixture was added with saturated brine, and extracted twice with dichloromethane. The organic layer was dried over sodium sulfate, and then the solvent was removed. The resulting residue was purified by silica gel column chromatography to obtain a phosphoric acid triester compound (4.87 g, 68%).
¹H-NMR (400MHz, CDCl₃) δ : 7.90-7.82 (2H, m) 7.76-7.69 (2H, m) 5.23 (1H, quin) 4.33 (1H, ddd) 4.20-4.09 (5H, m) 3.90 (2H, t) 3.76 (2H, t) 3.72 (3H, dd) 3.69 (2H, t) 2.31 (4H, q) 1.65-1.56 (4H, m) 1.33-1.22 (48H, m) 0.88 (6H, t)

The aforementioned phosphoric acid triester compound (3.37 g) was dissolved in 2-butanone (45 mL), and added with sodium iodide (2.86 g), and the mixture was refluxed by heating for 30 minutes. The mixture was left to cool, then added with 1 N hydrochloric acid, and extracted twice with a mixture of chloroform/methanol (5/1). The resulting organic layer was dried over sodium sulfate, and then the solvent was removed. The residue was added with acetonitrile, and the resulting crystals were separated by filtration, and dried to obtain a phosphoric acid diester compound (2.90 g, 87%). ¹H-NMR (400MHz, CDCl₃) δ : 7.90-7.82 (2H, m) 7.76-7.69 (2H, m) 5.23 (1H, quin) 4.33 (1H, dd) 4.20-4.09 (5H, m) 3.90 (2H, t) 3.76 (2H, t) 3.69 (2H, t) 2.31 (4H, q) 1.67-1.55 (4H, m) 1.35-1.21 (48H, m) 0.88 (6H, t)

The aforementioned phosphoric acid diester compound (3.98 g) was dissolved in dichloromethane (45 mL), and the solution was added with methanol (9 mL) and 40% aqueous methylamine (1.15 mL). The reaction mixture was stirred at room temperature for 12 hours, and then further added with 40% aqueous methylamine (0.38 mL), and stirring was continued at 40°C for 1 day. The solvent was evaporated, and the residue was added with acetonitrile. The resulting crystals were separated by filtration, washed again with acetonitrile, and purified by silica gel column chromatography to obtain an amine of the phosphoric acid diester compound (1.67 g, 49%).
¹H-NMR (300MHz, CDCl₃) δ : 5.23-5.15 (1H, m) 4.35 (1H, dd) 4.14 (1H, dd) 4.07-3.97 (2H, m) 3.93 (2H, t) 3.78-3.63 (4H, m) 3.05 (2H, bs) 2.29 (4H, dt) 1.67-1.55 (4H, m) 1.35-1.21 (48H, m) 0.88 (6H, t)

Diethylenetriaminepentaacetic acid dianhydride (182 mg) was added with DMF (30 mL), and further added with triethylamine (0.67 mL), and the mixture was stirred at 50°C for 2 hours. The aforementioned amine of phosphoric acid diester compound (350 mg) was dissolved in chloroform (5 mL), and added dropwise to the reaction mixture over 10 minutes. Stirring of the reaction mixture was continued at the same temperature, 50°C, for 1 hour, and the reaction mixture was added with water (1.5 mL), and stirred at room temperature. After the solvent was evaporated, the residue was added with 1 N hydrochloric acid, and extracted twice with a mixture of chloroform/methanol (5/1). After the resulting organic layer was dried over sodium sulfate, the solvent was removed. The residue was added with acetonitrile, and the resulting crystals were separated by filtration, washed again with acetonitrile, and dried to obtain Compound 1-5 (0.47 g, 88%).
Mass (MALDI-TOFF): m/z (α-cyano-4-hydroxycinnamic acid) 1109 (M-H)

Gadolinium(III) chloride (81 mg) was dissolved in methanol (3.5 mL), and the solution was added dropwise to a solution of Compound 1-5 (0.31 g) dissolved in methanol (10 mL). The mixture was added with water (3.8 mL), and then adjusted to pH 6 with 1 N aqueous sodium hydroxide. The precipitated crystals were separated by filtration, washed twice with acetonitrile and twice with methanol, and then dried to obtain a gadolinium complex of Compound 1-5 (0.24 g, 65%).

### Synthesis of Compound 2-1

2-[2-(2-Chloroethoxy)ethoxy]ethanol (11.0 g) and phthalimide potassium salt (11.2 g) were dissolved in N,N-dimethylformamide (DMF, 10 mL), and the solution was stirred at 120°C for 3 hours. The reaction mixture was left to cool, then added with dichloromethane, and washed 4 times with water and once with saturated brine. The resulting organic layer was dried over anhydrous magnesium sulfate, and then the solvent was removed. The resulting residue was purified by silica gel column chromatography to obtain a phthalimidated alcohol (13.9 g, 83%).
¹H-NMR (400MHz, CDCl₃) δ : 7.90-7.82 (2H, m) 7.76-7.69 (2H, m) 3.92 (2H, t) 3.76 (2H, t) 3.70-3.59 (6H, m) 3.55-3.52 (2H, m) 2.48 (1H, t)

The above alcohol (2.99 g) was dissolved in dichloromethane (20 mL), and the solution was added with triethylamine (TEA, 3.25 g) and stirred at 0°C. The reaction mixture was added with N,N-diisopropylmethylphosphoamidic chloride (2.70 mL), stirred at room temperature for 15 minutes, added with ice-cooled saturated aqueous sodium hydrogencarbonate, and extracted twice with dichloromethane. The organic layer was dried over sodium sulfate, and then the solvent was removed. The residue was added with dichloromethane (80 mL) and dissolved therein, and the solution was added with the above 1,2-dipalmitoyl glyceride (5.46 g) and tetrazole (0.97 g). The mixture was stirred for 30 minutes, and then added with 31% aqueous hydrogen peroxide (4 mL), and stirring was continued for 20 minutes. The reaction mixture was added with saturated brine, and extracted twice with dichloromethane. The organic layer was dried over sodium sulfate, and then the solvent was removed. The resulting residue was purified by silica gel column chromatography to obtain a phosphoric acid triester compound (7.40 g, 83%).
¹H-NMR (400MHz, CDCl₃) δ : 7.87-7.82 (2H, m) 7.75-7.69 (2H, m) 5.23 (1H, quin) 4.34 (1H, ddd) 4.22-4.08 (5H, m) 3.90 (2H, t) 3.76 (3H, dd) 3.74 (2H, t) 3.68-3.59 (6H, m) 2.31 (4H, q) 1.65-1.56 (4H, m) 1.33-1.22 (48H, m) 0.88 (6H, t)

The above phosphoric acid triester compound (3.71 g) was dissolved in 2-butanone (45 mL), and added with sodium iodide (3.00 g), and the mixture was refluxed by heating for 30 minutes. The mixture was left to cool, then added with 1 N hydrochloric acid, and extracted twice with a mixture of chloroform/methanol (5/1). The resulting organic layer was dried over sodium sulfate, and then the solvent was removed. The residue was added with acetonitrile and cooled on ice, and the resulting crystals were separated by filtration, washed with ice-cooled acetonitrile and dried to obtain a phosphoric acid diester compound (3.92 g, quantitative).
¹H-NMR (400MHz, CDCl₃) δ : 7.87-7.82 (2H, m) 7.75-7.69 (2H, m) 5.23 (1H, quin) 4.34 (1H, ddd) 4.22-4.08 (5H, m) 3.90 (2H, t) 3.76 (2H, t) 3.68-3.59 (6H, m) 2.31 (4H, q) 1.65-1.56 (4H, m) 1.33-1.22 (48H, m) 0.88 (6H, t)

The above phosphoric acid diester compound (3.92 g) was dissolved in dichloromethane (40 mL), and added with methanol (8 mL) and 40% aqueous methylamine (1.0 mL), and the mixture was stirred at room temperature for 15 hours. Then, the reaction mixture was further added with 40% aqueous methylamine (0.33 mL), and stirring was continued at 40°C for 6 hours. The solvent was evaporated, and the residue was added with acetonitrile. The resulting crystals were separated by filtration, washed with acetonitrile, and purified by silica gel column chromatography to obtain an amine of the phosphoric acid diester compound (1.28 g, 41%).
¹H-NMR (400MHz, CDCl₃) δ : 5.21 (1H, quin) 4.38 (1H, dd) 4.16 (1H, dd) 4.05-3.93 (4H, m) 3.80 (2H, bt) 3.70-3.60 (6H, m) 3.11 (2H, bs) 2.29 (4H, dt) 1.65-1.56 (4H, m) 1.33-1.22 (48H, m) 0.88 (6H, t)

Diethylenetriaminepentaacetic acid dianhydride (189 mg) was added with DMF (30 mL), and further added with triethylamine (0.70 mL), and the mixture was stirred at 50°C for 1 hour. The above amine of phosphoric acid diester compound (0.39 g) was dissolved in chloroform (5 mL), and added dropwise to the reaction mixture over 10 minutes. Stirring of the reaction mixture was continued at the same temperature, 50°C, for 30 minutes, and the reaction mixture was added with water (1.5 mL) and stirred at room temperature. After the solvent was evaporated, the residue was added with 1 N hydrochloric acid, and extracted twice with a mixture of chloroform/methanol (5/1). After the resulting organic layer was dried over sodium sulfate, the solvent was removed. The residue was added with acetonitrile, and the resulting crystals were separated by filtration, washed again with acetonitrile, and dried to obtain Compound 2-1 (0.51 g, 88%).

### Compound 2-1

### Mass (MALDI-TOFF): m/z (α -cyano-4-hydroxycinnamic acid) 1153 (M-H)

Gadolinium(III) chloride (128 mg) was dissolved in methanol (5.5 mL), and the solution was added dropwise to a solution of Compound 2-1 (0.51 g) dissolved in methanol (15 mL). The mixture was added with water (6.0 mL), and then adjusted to pH 6 with 1 N aqueous sodium hydroxide. The precipitated crystals were separated by filtration, washed once with a small volume of water, twice with acetonitrile and twice with methanol, and then dried to obtain a gadolinium complex of Compound 2-1 (0.44 g, 74%).

### Test Example 1: Preparation of liposomes

According to the method described in J. Med. Chem., 25 (12), 1500 (1982), dipalmitoyl-PC (Funakoshi, No. 1201-41-0225), dipalmitoyl-PS (Funakoshi, No. 1201-42-0237), and the compound of the present invention in the ratio mentioned below were dissolved in chloroform contained in an eggplant-shaped flask to form a uniform solution, and then the solvent was evaporated under reduced pressure to form a thin membrane on the bottom of the flask. The thin membrane was dried in vacuo, then added with an appropriate volume of 0.9% physiological saline (Hikari Pharmaceutical, No. 512) and ultrasonicated (probe type oscillator, Branson, No. 3542, 0.1 mW) for 5 minute with ice cooling to obtain a uniform liposome dispersion. Size of the particles contained in the resulting dispersion was measured by using WBC analyzer (Nihon Kohden, A-1042). The particle sizes were 85 to 110 nm. When the known comparative compound was added, liposomes were not formed with the following composition of phospholipids. Thus, it is clearly understood that the compound of the present invention can be efficiently incorporated in liposomes of the following composition, and has superior properties as a component lipid of liposomes for contrast medium.

### Maximum amount of formed liposomes

PC 50 nmol + PS 50 nmol + Gd complex of Compound 1-5 10 nmol
PC 50 nmol + PS 50 nmol + Comparative Compound 0 nmol

### (Comparative Compound)

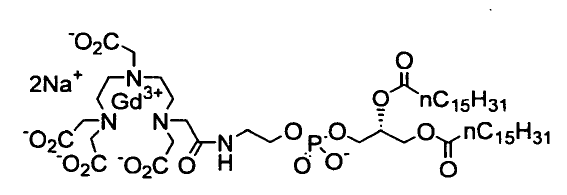

### Test Example 2: Uptake amount of iodine atoms in vascular smooth muscle cells

The above liposome preparation prepared by the method of Test Example 1 was added to the mixed culture system of vascular smooth muscle cells and macrophages described in International Patent Publication WO01/82977, and the cells were cultured at 37°C for 24 hours under 5% CO₂. Then, iodine atoms taken up into the vascular smooth muscle cells were quantified. From the results mentioned below, it is clearly understood that the compound of the present invention allows efficient uptake into vascular smooth muscle cells, and has superior properties as a component lipid of liposomes for contrast medium.

### Uptake amount

PC 50 nmol + PS 50 nmol + Gd complex of Compound 1-5 10 nmol: 6.8 nmol/mg protein
PC 50 nmol + PS 50 nmol + Comparative Compound 0 nmol: 0.0 nmol/mg protein

### Test Example 3: Toxicity test by continuous administration for 3 days in mice

Six-week old ICR male mice (Charles River Japan) were purchased, and after quarantine for 1 week, acclimatized for 1 week in a clean animal cage (air-conditioning: HEPA filter of class 1000, room temperature: 20 to 24°C, humidity: 35 to 60%). Then, in order to obtain the MTD value, a mouse serum suspension was given from the caudal vein. The mouse serum suspension was given by using physiological saline (Hikari Pharmaceutical) or a glucose solution (Otsuka Pharmaceutical) as a solvent. On the basis of the MTD value obtained, the mouse serum suspension was given everyday from the caudal vein for three consecutive days in an amount corresponding to 1/2 of the MTD value (n=3). The symptoms were observed up to 6 hours after each administration to observe neurotoxicity, and then autopsy was performed to examine major organs. It was confirmed that the compounds of the present invention show low toxicity and no neurotoxicity. Thus, it is clearly understood that the compounds of the present invention have superior characteristics as a component lipid of liposomes for a contrast medium for MRI.
Compound: MTD (mg/kg): neurotoxicity ("-" indicates negative for neurotoxicity, and "+" indicate positive for neurotoxicity)
Gadolinium complex of Compound 1-5: 100 mg/kg: -
Gadolinium complex of Compound 2-1: 100 mg/kg: -

### Test Example 4: Imaging test (WHHL rabbit)

According to a commonly used method, a liposome formulation (size: 85 to 120 nm) containing PC : PS : Gd complex of Compound 1-5 at 50 : 50 : 10 (nmol) was prepared by using a chloroform solution of PC (dipalmitoylphosphatidylcholine, Funakoshi, No. 850355C), PS (dipalmitoylphosphatidylserine, Funakoshi, No. 840037C), and Compound 1-5. For preparation of the formulation, distilled water for injection (Ohtsuka Pharmaceutical) was used.

A 12 months-old WHHL rabbit with an already formed pathological lesion in the aortic arch (Kitayama LEBS) was obtained and acclimatized for 1 week. The above liposome formulation was administered from the subaural vein (at 80 mg/kg as an amount of the Gd complex of Compound 1-5), and after 15 minutes, NRI imaging was performed targeting to the pathological lesion in the arch.
Conditions for the imaging (photographed using Vioview)
MRI: Varian Unity INOVA 4.7 T
Pulse sequence: ECG triggered Spin-echo multislice method, TE/TR=13/500 msec
Size of imaging: 15 x 15 cm
Slice thickness: 2 mm
Integration number: 2 times

Results are shown in Fig. 1. As compared to the photograph before the administration (upper figure), imaging of the pathological lesion was observed with white gleam of the arteriosclerotic lesion formed in the aortic wall in the photograph at 15 minutes after the administration (lower figure).

## Claims

1. A compound represented by formula (1) or (2), wherein in formula (1) R¹ and R² are selected from the following group:
1) R¹=R²=n-C₈H₁₇
2) R¹=R²=n-C₁₀H₂₁
4) R¹=R²=n-C₁₃H₂₇
5) R¹=R²=n-C₁₅H₃₁
6) R¹=R²=n-C₁₇H₃₅
7) R¹=R²=n-C₂₀H₄₁
8) R¹=R²=n-C₂₅H₅₁
9) R¹=R²=n-C₃₀H₆₁
10) R¹=n-C₁₃H₂₇,
R²=n-C₁₅H₃₁
and wherein in formula (2) p and n are selected from the following group:
1) p=1, n=3
2) p=1, n=4
3) p=1, n=5
4) p=2, n=2
5) p=2,n =3
6) p=2, n=4
7) p=n, n=5
and wherein formula (1) and (2) are as follows:

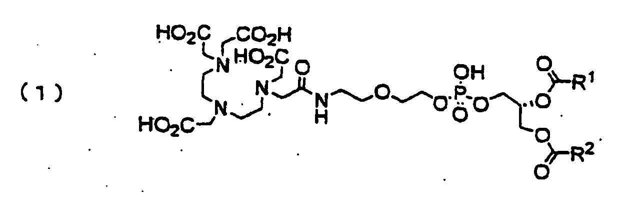

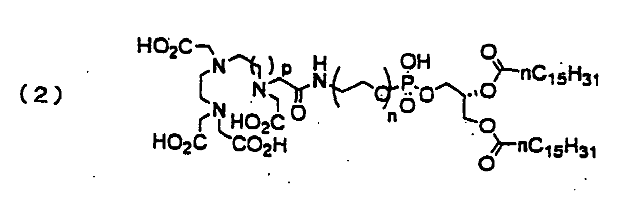

2. A chelate compound or a salt thereof, which consists of the compound or a salt thereof according to claim 1 and a metal ion.

3. A chelate compound or a salt thereof according to claim 2, wherein the metal ion is a metal ion of an element selected from elements of atomic numbers 21 to 29, 31, 32, 37 to 39, 42 to 44, 49, and 57 to 83.

4. The chelate compound or a salt thereof according to claim 2, wherein the metal ion is a metal ion of a paramagnetic element selected from elements of atomic numbers 21 to 29, 42, 44, and 57 to 71.

5. A liposome containing the compound or salt thereof according to any one of claims 1 to 4 as a membrane component.

6. The liposome according to claim 5, which contains a phosphatidylcholine and a phosphatidylserine as membrane components.

7. A contrast medium for MRI, which comprises the liposome according to claim 5 or 6.

8. The contrast medium for MRI according to claim 7, which is used for imaging of a vascular disease.

9. The contrast medium for MRI according to claim 7 or 8, which is used for imaging of vascular smooth muscle cells abnormally proliferating under influence of foam macrophages.

10. The contrast medium for MRI according to any one of claims 7 to 9, which is used for imaging of a tissue or lesion in which macrophages localize.

11. The contrast medium for MRI according to claim 10, wherein the tissue in which macrophages localize is selected from the group consisting of tissues of liver, spleen, air vesicle, lymph node, lymph vessel, and renal epithelium.

12. The contrast medium for MRI according to claim 10, wherein the lesion in which macrophages localize is selected from the group consisting of lesions of tumor, inflammation, and infection.

13. A contrast medium for scintigraphy, which comprises the liposome according to claim 5 or 6.

14. The contrast medium for scintigraphy according to claim 13, which is used for imaging of a vascular disease.

15. The contrast medium for scintigraphy according to claim 13 or 14, which is used for imaging of vascular smooth muscle cells abnormally proliferating under influence of foam macrophages.

16. The contrast medium for scintigraphy according to any one of claims 13 to 15, which is used for imaging of a tissue or lesion in which macrophages localize.

17. The contrast medium for scintigraphy according to claim 16, wherein the tissue in which macrophages localize is selected from the group consisting of tissues of liver, spleen, air vesicle, lymph node, lymph vessel, and renal epithelium.

18. The contrast medium for scintigraphy according to claim 16, wherein the lesion in which macrophages localize is selected from the group consisting of lesions of tumor, inflammation, and infection.

## Patentansprüche

1. Verbindung, dargestellt durch die Formel (1) oder (2), worin in Formel (1) R¹ und R² ausgewählt sind aus der folgenden Gruppe:
1) R¹=R²=n-C₈H₁₇
2) R¹=R²=n-C₁₀H₂₁
4) R¹=R²=n-C₁₃H₂₇
5) R¹=R²=n-C₁₅H₃₁
6) R¹=R²=n-C₁₇H₃₅
7) R¹=R²=n-C₂₀H₄₁
8) R¹=R²=n-C₂₅H₅₁
9) R¹=R²=n-C₃₀H₆₁
10) R¹=n-C₁₃H₂₇,
R²=n-C₁₅H₃₁
und worin in Formel (2) p und n ausgewählt sind aus der folgenden Gruppe:
1) p=1, n=3
2) p=1, n=4
3) p=1, n=5
4) p=2, n=2
5) p=2, n=3
6) p=2, n=4
7) p=2, n=5,
und worin Formel (1) und (2) wie folgt sind:

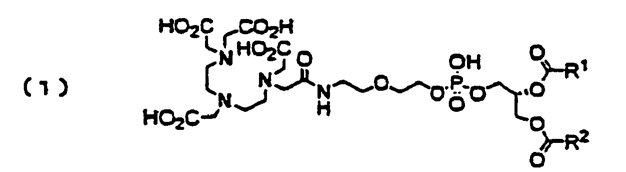

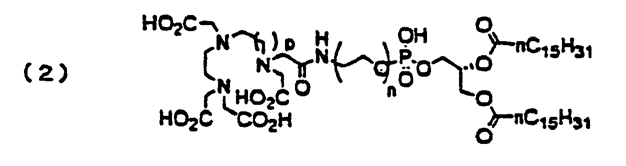

2. Chelatverbindung oder ein Salz hiervon, welche aus der Verbindung oder einem Salz hiervon gemäß Anspruch 1 und einem Metallion besteht.

3. Chelatverbindung oder ein Salz hiervon gemäß Anspruch 2, worin das Metallion ein Metallion eines Elements ist, das ausgewählt aus Elementen mit den Ordnungszahlen 21 bis 29, 31, 32, 37 bis 39, 42 bis 44, 49 und 57 bis 83.

4. Chelatverbindung oder ein Salz hiervon gemäß Anspruch 2, worin das Metallion ein Metallion eines paramagnetischen Elements ist, das ausgewählt ist aus Elementen der Ordnungszahlen 21 bis 29, 42, 44 und 57 bis 71.

5. Liposom, enthaltend die Verbindung oder ein Salz hiervon gemäß irgendeinem der Ansprüche 1 bis 4 als eine Membrankomponente.

6. Liposom gemäß Anspruch 5, welches ein Phosphatidylcholin und ein Phosphatidylserin als Membrankomponenten umfasst.

7. Kontrastmittel für MRI, welches das Liposom gemäß Anspruch 5 oder 6 umfasst.

8. Kontrastmittel für MRI gemäß Anspruch 7, welches für die Bebilderung einer vaskulären Krankheit verwendet wird.

9. Kontrastmittel für MRI gemäß Anspruch 7 oder 8, welches für die Bebilderung von vaskulären glatten Muskelzellen verwendet wird, die unter dem Einfluss von Schaummakrophagen abnormal wuchern.

10. Kontrastmittel für MRI gemäß irgendeinem der Ansprüche 7 bis 9, welches für die Bebilderung eines Gewebes oder einer Läsion verwendet wird, worin Makrophagen lokalisiert sind.

11. Kontrastmittel für MRI gemäß Anspruch 10, worin das Gewebe, in dem Makrophagen lokalisiert sind, ausgewählt ist aus der Gruppe bestehend aus Geweben der Leber, der Milz, von Luftvesikeln, Lymphknoten, Lymphgefäßen und dem renalen Epithelium.

12. Kontrastmittel für MRI gemäß Anspruch 10, worin die Läsion, worin Makrophagen lokalisiert sind, ausgewählt ist aus der Gruppe bestehend aus Läsionen von Tumoren, Entzündungen und Infektionen.

13. Kontrastmittel für die Szintigraphie, welches ein Liposom gemäß Anspruch 5 oder 6 umfasst.

14. Kontrastmittel für die Szintigraphie gemäß Anspruch 13, das für die Bebilderung einer vaskulären Krankheit verwendet wird.

15. Kontrastmittel für die Szintigraphie gemäß Anspruch 13 oder 14, welches für die Bebilderung von vaskulären glatten Muskelzellen verwendet wird, die unter dem Einfluss von Schaummakrophagen abnormal wuchern.

16. Kontrastmittel für die Szintigraphie gemäß irgendeinem der Ansprüche 13 bis 15, welches für die Bebilderung eines Gewebes oder einer Läsion, worin Makrophagen lokalisiert sind, verwendet wird.

17. Kontrastmittel für die Szintigraphie gemäß Anspruch 16, worin das Gewebe, worin Makrophagen lokalisiert sind, ausgewählt ist aus der Gruppe bestehend aus Geweben der Leber, der Milz, der Luftvesikel, der Lymphknoten, der Lymphgefäße und dem renalen Epithelium.

18. Kontrastmittel für die Szintigraphie gemäß Anspruch 16, worin die Läsion, worin Makrophagen lokalisiert sind, ausgewählt ist aus der Gruppe bestehend aus Läsionen von Tumoren, Entzündungen und Infektionen.

## Revendications

1. Composé représenté par la formule (1) ou (2), dans lequel, dans la formule (1), R¹ et R² sont sélectionnés à partir du groupe suivant :
1) R¹ = R² n-C₈H₁₇
2) R¹ = R² n-C₁₀H₂₁
4) R¹ = R² n-C₁₃H₂₇
5) R¹ = R² n-C₁₅H₃₁
6) R¹ = R² n-C₁₇H₃₅
7) R¹ = R² = n-C₂₀H₄₁
8) R¹ = R² = n-C₂₅H₅₁
9) R¹ = R² = n-C₃₀H₆₁
10) R¹ = n-C₁₃H₂₇
R² = n-C₁₅H₃₁
et dans lequel, dans la formule (2), p et n sont sélectionnés à partir du groupe suivant :
1) p = 1, n = 3
2) p = 1, n = 4
3) p = 1, n = 5
4) p = 2, n = 2
5) p = 2, n = 3
6) p = 2, n = 4
7) p = 2, n = 5.
et dans lequel les formules (1) et (2) sont comme suit :

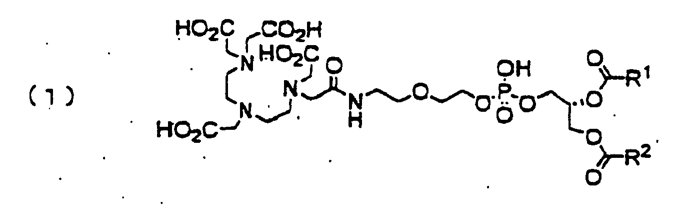

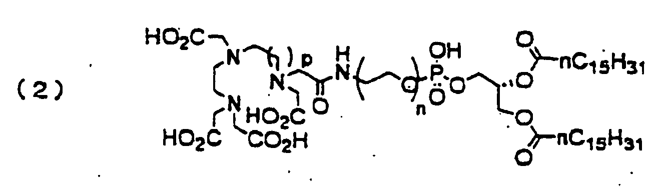

2. Composé de chélate ou sel de celui-ci, qui est constitué par le composé ou un sel de celui-ci selon la revendication 1 et un ion de métal.

3. Composé de chélate ou sel de celui-ci selon la revendication 2, dans lequel l'ion de métal est un ion de métal d'un élément sélectionné à partir d'éléments de numéros atomiques 21 à 29, 31, 32, 37 à 39, 42 à 44, 49, et 57 à 83.

4. Composé de chélate ou sel de celui-ci selon la revendication 2, dans lequel l'ion de métal est un ion de métal d'un élément paramagnétique sélectionné à partir d'éléments de numéros atomiques 21 à 29, 42, 44, et 57 à 71.

5. Liposome contenant le composé ou le sel de celui-ci selon l'une quelconque des revendications 1 à 4 en tant que composant de membrane.

6. Liposome selon la revendication 5, qui contient une phosphatidylcholine et une phosphatidylsérine en tant que composants de membrane.

7. Produit de contraste pour IRM, qui comprend le liposome selon la revendication 5 ou 6.

8. Produit de contraste pour IRM selon la revendication 7, qui est utilisé pour l'imagerie d'une maladie vasculaire.

9. Produit de contraste pour IRM selon la revendication 7 ou 8, qui est utilisé pour l'imagerie de cellules musculaires lisses vasculaires proliférant anormalement sous l'influence de macrophages mousseux.

10. Produit de contraste pour IRM selon l'une quelconque des revendications 7 à 9, qui est utilisé pour l'imagerie d'un tissu ou d'une lésion dans lequel des macrophages se localisent.

11. Produit de contraste pour IRM selon la revendication 10, dans lequel le tissu dans lequel des macrophages se localisent est sélectionné à partir du groupe constitué par de tissus du foie, de la rate, de sac alvéolaire, de noeud lymphoïde, de vaisseau lymphatique et d'épithélium rénal.

12. Produit de contraste pour IRM selon la revendication 10, dans lequel la lésion dans laquelle des macrophages se localisent est sélectionnée à partir du groupe constitué par des lésions de tumeur, d'inflammation, et d'infection.

13. Produit de contraste pour scintigraphie, qui comprend le liposome selon la revendication 5 ou 6.

14. Produit de contraste pour scintigraphie selon la revendication 13, qui est utilisé pour l'imagerie d'une maladie vasculaire.

15. Produit de contraste pour scintigraphie selon la revendication 13 ou 14, qui est utilisé pour l'imagerie de cellules musculaires lisses vasculaires proliférant anormalement sous l'influence de macrophages mousseux.

16. Produit de contraste pour scintigraphie selon l'une quelconque des revendications 13 à 15, qui est utilisé pour l'imagerie d'un tissu ou d'une lésion dans laquelle des macrophages se localisent.

17. Produit de contraste pour scintigraphie selon la revendication 16, dans lequel le tissu dans lequel des macrophages se localisent est sélectionné à partir du groupe constitué par des tissus du foie, de la rate, de sac alvéolaire, du noeud lymphoïde, de vaisseau lymphatique et d'épithélium rénal.

18. Produit de contraste pour scintigraphie selon la revendication 16, dans lequel la lésion dans laquelle des macrophages se localisent est sélectionnée à partir du groupe constitué par des lésions de tumeur, d'inflammation et d'infection.
